# EUROPÄISCHE PATENTSCHRIFT

(11) **EP 1 088 565 B1**
(45) Veröffentlichungstag und Bekanntmachung des Hinweises auf die Patenterteilung: **11.04.2001**
(21) Anmeldenummer: 99118684.2
(22) Anmeldetag: 22.09.1999
(51) Int. Cl.: A61M 5/00

(54) **Verschweisste Sterilverpackung mit einem Set für die intravesikale Instillation**
Sealed sterile package with pack for intravesical instillation
Emballage scellé avec pacquet pour instillation intravésical

(43) Veröffentlichungstag der Anmeldung: 04.04.2001
(73) Patentinhaber: Stegemann, Klaus, 21035 Hamburg (DE); Martens, Torsten, 21037 Hamburg (DE)
(72) Erfinder: Stegemann, Klaus, 21035 Hamburg (DE); Martens, Torsten, 21037 Hamburg (DE)
(74) Vertreter: Fleck, Thomas, Dr. Dipl.-Chem.

(56) Entgegenhaltungen:
- EP-A- 0 925 798
- WO-A-99/01069
- US-A- 3 507 386
- US-A- 4 171 699
- US-A- 4 177 620
- US-A- 5 376 081
- HEINICKE H -J: "STERILHALTUNG VON MEDIZINISCHEN INSTRUMENTEN IN PLASTBEUTELN" ZEITSCHRIFT FUER AERZTLICHE FORTBILDUNG,DD,JENA, Bd. 60, 1. Mai 1966 (1966-05-01), Seite 581-583 XP000083240 ISSN: 0044-2178

## Beschreibung

Die vorliegende Erfindung betrifft eine sterile Spritzen-Verpackung nach dem Oberbegriff des Anspruches 1.

Eine derartige sterile Spritzen-Verpackung ist aus der WO 99/01069 A bekannt.

Die Erfindung beschäftigt sich ferner mit dem Gebiet der intravesikalen Instillation bei Harndrang-Inkontinenz infolge einer Dysfunktion des Detrusors. Hierzu sind schon unterschiedliche Arzneimittelwirkstoffe eingesetzt worden. Das Problem bestand darin, daß die Patienten entweder gezwungen waren, entweder eine sterilisierte Instillationslösung selbst auf eine Spritze aufzuziehen und mittels eines Adapters die Verbindung zum Katheter herzustellen und zu instillieren, oder eine nicht endsterilisierte fertige Spritze mit einem Adapter zu verbinden und an den Katheter anzuschließen, was eine sehr kurze Haltbarkeit von einigen Wochen und ein erhöhtes Kontaminationsrisiko mit sich bringt. Auch war es problematisch, eine Tablette oder dgl. mit dem entsprechenden Arzneimittelwirkstoff in einer wässrigen Lösung aufzulösen, dann die entstandene Lösung in die Spritze aufzuziehen und letztendlich zu applizieren. Auch hier gab es immer wieder Infektionen und Komplikationen für den Patienten. Ein weiteres Problem des Standes der Technik lag in der Lichtempfindlichkeit verschiedener Arzneimittelwirkstoffe, insbesondere des Oxybutynins, die ein Aufziehen des Arzneimittelwirkstoffes in die Spritze erst unmittelbar vor seiner Applikation zuließ.

Der Erfindung liegt deshalb die Aufgabe zugrunde, ein Set zu konzipieren, das einem an einer medikamentös regulierbaren Dysfunktion des Detrusors leidenden Patienten eine sichere, einfache und schnelle intravesikale Instillation zu ermöglichen, bei welcher das Risiko der Infektion minimiert wird, und gleichzeitig eine möglichst lange Lagerung der abbauempfindlichen Arzneimittelwirkstoffe zu gewährleisten, ohne daß der Patient mit diesen bei der Applikation in Berührung kommt.

Überraschender Weise wird diese Aufgabe und das Ziel durch die verschweißte Verpackung gemäß Anspruch 1 gelöst. Danach weist die verschweißte Verpackung einen vollständigen Set für die intravesikale Instillation auf, der nach Öffnen der Verpackung und Abnehmen der Verschlußkappe sofort gebrauchsfertig an einen Katheter angeschlossen werden kann. Letzteres kann sicher, einfach und schnell durch den Patienten selbst durchgeführt werden, der nicht auf die Hilfe eines Arztes hierzu angewiesen ist. Das besondere an dem Set ist die Tatsache, daß die eingesetzte Einmalspritze schon die Arzneimittelwirkstofflösung enthält. Hierzu bedurfte es der Sterilisation, insbesondere der Dampfsterilisation der Spritze und außerdem der Lichtundurchlässigkeit des Zylinders der Spritze, welcher eingefärbt ist, d.h. opak ist. Lichtempfindliche Arzneimittelwirkstoffe werden hierdurch geschützt. Ebenso verhindert der latexfreie Dichtungsring am vorderen Kolbenende etwaige Reaktionen mit dem Arzneimittelwirkstoff. Erfindungsgemäß sind also sämtliche Einzelteile des Sets schon sterilisiert und für den Gebrauch zur Instillation zusammengesetzt, was es bisher noch nicht gegeben hat.

Im folgenden wird ein Ausführungsbeispiel im Zusammenhang mit der Zeichnung zur Erläuterung der Erfindung näher beschrieben. Die Erfindung ist jedoch nicht auf dieses Ausführungsbeispiel beschränkt, sondern läßt sich durch den Fachmann im Rahmen der Ansprüche modifizieren. Es zeigt:
eine Schemaskizze der erfindungsgemäßen verschweißten Verpackung mit einem Set für die intravesikale Instillation.

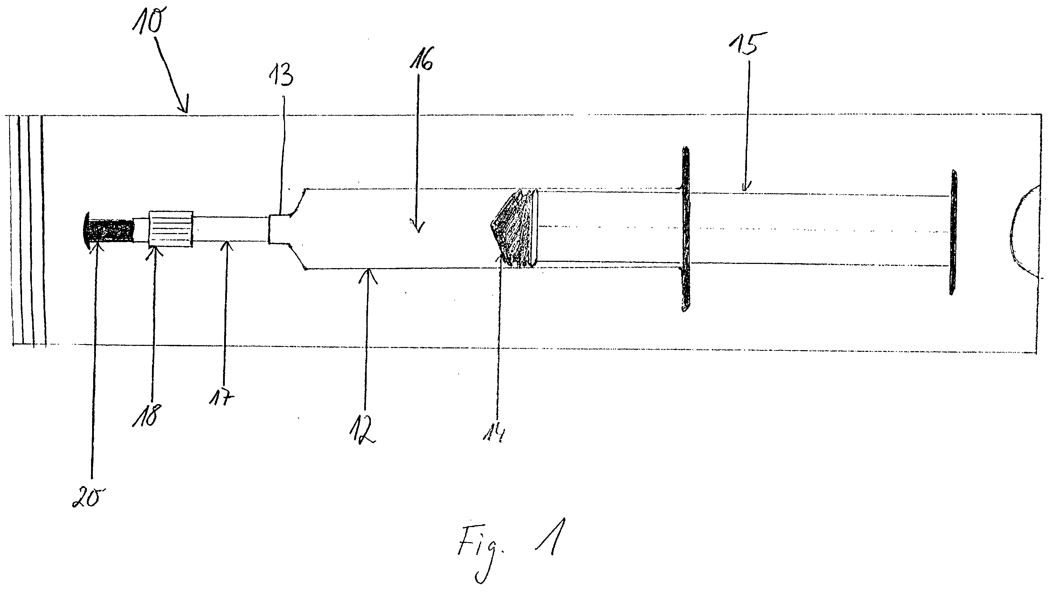

In Fig. 1 ist die erfindungsgemäße verschweißte Verpackung allgemein mit 10 bezeichnet. Diese Verpackung 10 ist handelsüblich und umfaßt eine papierbeschichtete Kunststoff-Folie. Der Set in der Verpackung 10 ist vollständig und somit unmittelbar nach dem Öffnen der Verpackung gebrauchsfertig, nach dem die Verschlußkappe 20 vom Adapter 18 abgenommen wird. Der Adapter 18, bei dem es sich um ein Kegel- oder Stufenadapter handeln kann, wird unmittelbar an den Katheter (nicht gezeigt) angeschlossen. Letzteres kann somit schnell und einfach durch den Patienten (nicht gezeigt) selbst erfolgen, der auf die Hilfe eines Arztes nicht angewiesen ist und auch keinerlei Vorbereitungen mehr für die Bereitstellung der Arzneimittellösung treffen muß. Bei letzterer handelt es sich um eine isotonische Natriumchloridlösung 16 eines Arzneimittelwirkstoffes. Als Arzneimittelwirkstoffe können beispielsweise Oxybutynin, Trospiumchlorid oder Verapamil eingesetzt werden. Übliche Konzentrationen liegen im Bereich von 0,025 bis 0,1% Wirkstoff in isotonischer NaCl-Lösung. Wie aus der Schema-skizze ersichtlich, ist diese den arzneimittelwirkstoffenthaltende Natriumchloridlösung 16 schon in der Spritze aufgezogen, da der Kolben 15 im Zylinder 12 zurückgezogen angeordnet ist. Der Zylinder 12 besteht aus dem TPX-Kunststoff Methylpenten-Copolymer, der mit einem Farbstoff versetzt ist. Der Kolben 15 besteht aus Polypropylen und ist genauso wie der Zylinder 12 dampfsterilisierbar. Am vorderen Ende des Kolbens 15 ist ein latexfreier Dichtungsring oder Kolbenstopfen aus Isopren angeordnet, der eine schwarze Farbe besitzt. Das vordere Ende 13 des Zylinders 12 ist über ein Verbindungstück 17 mit dem Adapter 18 verbunden, auf dem zur Abdichtung eine Verschlußkappe 20 aus geeignetem Material wie Isopren aufgesetzt ist. Es dürfte einleuchten, daß ggf. auf das Verbindungsstück 17 verzichtet werden kann, sofern der Adapter 18 direkt mit dem vorderen Ende 13 des Zylinders 12 der Einmalspritze verbunden werden kann. Im vorliegenden Ausführungsbeispiel ist im opaken Zylinder 12 eine 30ml umfassende Oxybutynin-NaCl-Lösung 16 aufgezogen. Der mengenmäßige Umfang kann jedoch in geeignetem Rahmen verändert werden.

## Patentansprüche

1. Sterile Spritzen-Verpackung (10), umfassend eine sterilisierte Spritze mit einem Zylinder (12) und einem Dichtungselemen (14), gefüllt mit einer Flüssigkeit (16), und mit einem Adapter (18) und einer sterilisierten Verschlußkappe (20) versehen, dadurch gekennzeichnet, daß die Spritze eine Einmalspritze ist, und daß der Zylinder (12) der Spritze opak ist, und daß die Spritze einen Kolben mit dem Dichtungselement (14) an dessen vorderem Ende umfaßt, und daß das Dichtungselement (14) ein latexfreier Dichtungsring ist, und daß die Flüssigkeit eine isotonische Natriumchloridlösung eines Arzneimittelwirkstoffes ist.

2. Verschweißte Verpackung nach Anspruch 1, gekennzeichnet durch einen der folgenden Arzneimittelwirkstoffe: Oxybutynin, Trospiumchlorid, Verapamil.

3. Verschweißte Verpackung nach Anspruch 1 oder 2, dadurch gekennzeichnet, daß der Zylinder (12) der sterilisierten Einmalspritze aus einem TPX-Kunststoff, insbesondere Methylpenten-Copolymer mit einem Farbstoff, besteht, und daß der Kolben (15) aus Polypropylen besteht, die dampfsterilisierbar sind.

4. Verschweißte Verpackung nach Anspruch 1 bis 3, dadurch gekennzeichnet, daß der sterilisierte Adapter (18) ein Kegel- oder Stufenadapter ist.

5. Verschweißte Verpackung nach Anspruch 4, dadurch gekennzeichnet, daß der sterilisierte Adapter (18) einstückig mit der sterilisierten Einmalspritze ausgebildet ist, oder über ein Verbindungsstück (17) mit der Spritze verbunden ist.

6. Verschweißte Verpackung nach Anspruch 1 bis 5, dadurch gekennzeichnet, daß der Arzneimittelwirkstoff Oxybutynin als 0,025 bis 0,1% isotonische NaCl-Lösung vorgesehen ist.

7. Verschweißte Verpackung nach einem oder mehreren der vorstehenden Ansprüche, dadurch gekennzeichnet, daß die sterilisierte Einmalspritze 20 bis 40 ml der Arzneimittelwirkstofflösung enthält.

8. Verschweißte Verpackung nach Anspruch 1, dadurch gekennzeichnet, daß die sterilisierte Einmalspritze dampfsterilisiert ist.

## Claims

1. Sterile syringe pack (10) incorporating a sterilized syringe with a cylinder (12) and a sealing element (14) filled with a liquid (16) and having an adaptor (18), together with a sterilized closing cap (20), characterized in that the syringe is a disposable syringe and that the syringe cylinder (12) is opaque, that the syringe incorporates a plunger with the sealing element (14) at its front end, that the sealing element (14) is a latex-free sealing ring and that the liquid is an isotonic sodium chloride solution of a medicinal substance.

2. Welded pack according to claim 1, characterized by one of the following medicinal substances, oxybutynin, trospium chloride and verapamil.

3. Welded pack according to claim 1 or 2, characterized in that the cylinder (12) of the sterilized disposable syringe comprises a TPX plastic, particularly methyl pentene copolymer with a dye and that the plunger (15) comprises polypropylene, both of which are steam-sterilizable.

4. Welded pack according to one of the claims 1 to 3, characterized in that the sterilized adaptor (18) is a conical or step adaptor.

5. Welded pack according to claim 4, characterized in that the sterilized adaptor (18) is constructed in one piece with the sterilized disposable syringe or is connected to the syringe by means of a connecting piece (17).

6. Welded pack according to one of the claims 1 to 5, characterized in that the medicinal substance is oxybutynin in the form of a 0.025 to 0.1% isotonic NACl solution.

7. Welded pack according to one or more of the preceding claims, characterized in that the sterilized disposable syringe contains 20 to 40 ml of the medicinal substance.

8. Welded pack according to claim 1, characterized in that the sterilized disposable syringe is steam-sterilized.

## Revendications

1. Emballage de seringue stérile (10), comprenant une seringue stérilisée avec un cylindre (12) et un élément d'étanchéité (14), rempli d'un liquide (16), et avec un adaptateur (18) et un bouchon stérilisé (20), caractérisé en ce que la seringue est une seringue à usage unique, en ce que le cylindre (12) de la seringue est opaque, en ce que la seringue comprend un piston avec l'élément d'étanchéité (14) à son extrémité avant, en ce que l'élément d'étanchéité (14) est un anneau d'étanchéité sans latex et en ce que le liquide est une solution de chlorure de sodium isotonique d'une substance active médicamenteuse.

2. Emballage soudé selon la revendication 1, caractérisé par l'une des substances actives médicamenteuses suivantes : oxybutynine, chlorure de trospium, verapamil.

3. Emballage soudé selon la revendication 1 ou 2, caractérisé en ce que le cylindre (12) de la seringue stérile à usage unique est en une matière synthétique TPX, notamment en un copolymère de méthylpentène avec un colorant, et en ce que le piston (15) est en polypropylène, qui sont stérilisables à la vapeur.

4. Emballage soudé selon l'une des revendications 1 à 3, caractérisé en ce que l'adaptateur (18) stérilisé est un adaptateur conique ou à gradins.

5. Emballage soudé selon la revendication 4, caractérisé en ce que l'adaptateur (18) stérilisé est conçu d'un seul tenant avec la seringue stérilisée à usage unique ou est assemblé à la seringue avec une pièce d'assemblage (17).

6. Emballage soudé selon l'une des revendications 1 à 5, caractérisé en ce que la substance active médicamenteuse oxybutynine est prévue sous forme de solution de NaCl isotonique à 0,025 à 0,1%.

7. Emballage soudé selon une ou plusieurs des revendications précédentes, caractérisé en ce que la seringue stérilisée à usage unique contient 20 à 40 ml de la substance active médicamenteuse.

8. Emballage soudé selon la revendication 1, caractérisé en ce que la seringue stérilisée à usage unique est stérilisée à la vapeur.
